# EUROPEAN PATENT APPLICATION

(11) **EP 4 781 914 A1**
(43) Date of publication of application: **29.07.2026**
(21) Application number: 25153620.7
(22) Date of filing: 23.01.2025
(51) Int. Cl.: A61B 8/00

(54) **ULTRASOUND PROBE COOLING**

(71) Applicant: Koninklijke Philips N.V., 5656 AE Eindhoven (NL)
(72) Inventor: SUDOL, Wojtek, Eindhoven (NL)
(74) Representative: Philips Intellectual Property & Standards

(57) **Abstract**

The invention relates to an actively cooled ultrasound system. The system includes a cable (200) comprising a cable connector (250) with an inlet (215) and outlet (235) for coolant, a heat pump to draw the coolant, and an ultrasound probe (100) with an imaging sensor (120) and an electronic circuit (150). An open-loop cooling circuit circulates the coolant past the electronic circuit to absorb and transport heat away. The cable has a first coolant conduit (210) to transport coolant to the probe and a second coolant conduit (230) to transport coolant back to the connector.

## Description

### FIELD OF THE INVENTION

The invention relates to ultrasound imaging. In particular, to thermal management in ultrasound imaging probes.

### BACKGROUND OF THE INVENTION

Ultrasound probes are generally power-limited due to heat problems affecting image quality. Active fluid cooling with heat exchangers in the probe and fluid pipes through the cable exist (e.g., Siemens 4Zlc, see 4Z1c real-time volume imaging transducer whitepaper (June 2008), https://cdn0.scrvt.com/39b415fb07de4d9656c7b516d8e2d907/1800000000064735/81ce01e55d56/Whitep aper_4Z1c_1800000000064735.pdf). However, these systems are typically clunky, reducing operator satisfaction and resulting in operator injuries. Additionally, these systems are often unreliable due to leakage and other issues associated with liquid coolants. The complexity and maintenance requirements of liquid cooling systems further add to the overall system cost. There is a need for a simpler, more reliable cooling solution that can effectively manage heat generation in ultrasound probes, allowing for increased power consumption and improved performance without compromising safety or image quality.

### SUMMARY OF THE INVENTION

It is, inter alia, an object of the invention to provide to provide for a reliable active cooling solution for ultrasound probes. The invention is defined by the independent claims. Advantageous embodiments are defined in the dependent claims.

According to an embodiment, there is provided an ultrasound probe and cable assembly comprising:
- an ultrasound probe comprising
   - an imaging sensor; and
   - an electronic circuit configured to control the imaging sensor;
- a cable connector;
- a cable connecting the ultrasound probe and the cable connector, the cable comprising:
   - a plurality of conductors configured to transmit electrical signals between the cable connector and the ultrasound probe; and
- an open-loop cooling circuit comprising:
   - an inlet within the cable connector, the inlet configured to allow entry of a coolant to the cooling circuit;
   - an outlet within the cable connector, the outlet configured to allow exit of a coolant from the cooling circuit;
   - a heat exchanger section within the ultrasound probe, the heat exchanger section configured to circulate coolant past at least part of the electronic circuit
   - a first coolant conduit, the first coolant conduit configured to transport coolant from the inlet through the cable to the heat exchanger section;
   - a second coolant conduit, the second coolant conduit configured to transport coolant from the heat exchanger section to the outlet; and
   - a pump configured to propagate coolant through the cooling system;
wherein the coolant comprises a gas.

In an example, the second coolant conduit is formed by empty spaces inside a jacket of the cable between the plurality of conductors and the first coolant conduit.

In an example, at least a part of the electronic circuit is enclosed in the heat exchanger section.

In an example, the heat exchanger section comprises a thermal insulation layer, the thermal insulation layer configured to contain heat generated by the electronic circuit.

In an example, the ultrasound probe and cable assembly further comprising a temperature sensor for determining a temperature inside the ultrasound probe; and
wherein an intensity of the pump is dependent on the determined temperature.

In an example, the cable is further configured to dissipate heat transported by the coolant in the second conduit.

In an example, the jacket of the cable comprises a thermally conductive material.

In an example, the coolant is air.

In an example, the pump is placed at the inlet in the cable connector.

In an example, the cooling system comprises multiple pumps.

According to an embodiment there is provided a cable connector for an ultrasound probe and cable assembly, the cable connector comprising:
- a connector housing, the connector housing comprising:
   - an inlet, the inlet configured to allow entry of a coolant to the connector,
   - an outlet, the outlet configured to allow exit of the coolant from the connector,
   - a pump inside the connector housing and connected to the inlet, the heat pump configured to draw the coolant through the inlet and expel it through the outlet, wherein the coolant is a gas.

According to an embodiment there is provided an ultrasound probe, the ultrasound probe comprising:
- an imaging sensor;
- an electronic circuit configured to control the imaging sensor; and
- an open-loop cooling circuit configured to circulate coolant past at least a part of the electronic circuit.

According to an embodiment there is provided a cable for an ultrasound probe and cable assembly, the cable comprising:
- a plurality of conductors configured to transmit electrical signals;
- a first coolant conduit configured to transport coolant from a connector to an ultrasound probe; and
- a second coolant conduit configured to transport coolant from the ultrasound probe to the connector, wherein the coolant is a gas.

According to an embodiment there is provided a method of cooling an ultrasound probe, the method comprising:
- drawing coolant through an inlet in a cable connector;
- transporting the coolant from the cable connector to an ultrasound probe via a cable;
- circulating the coolant past at least a part of an electronic circuit inside the ultrasound probe, wherein the coolant is configured to absorb heat generated by the electronic circuit;
- transporting the coolant from the ultrasound probe back to the cable connector via the cable;
- expelling the coolant from the cable connector through an outlet in the cable connector.

In an example of the method,
- the coolant is drawn through the inlet in the cable connector by a heat pump;
- the coolant is transported from the cable connector to the ultrasound probe via a first coolant conduit in the cable;
- the coolant is circulated past at least part of the electronic circuit through a cooling circuit inside the ultrasound probe; and
- the coolant is transported from the ultrasound probe to the cable connector via a second coolant conduit in the cable.

These and other aspects of the invention will be apparent from and elucidated with reference to the embodiments described hereinafter.

### BRIEF DESCRIPTION OF THE DRAWINGS

Fig. 1 shows a schematic of an exemplary ultrasound system.
Fig. 2 shows a schematic of an exemplary ultrasound probe.
Fig. 3 shows a schematic of an exemplary ultrasound cable.
Fig. 4 shows a schematic of an exemplary method for cooling an ultrasound probe.

### DETAILED DESCRIPTION OF EMBODIMENTS

The invention relates to an actively cooled ultrasound system. In particular, the ultrasound system comprises an inlet of a coolant at a cable connector, wherein the coolant is transported to an ultrasound probe through a cable connecting the cable connector and the ultrasound probe. Within the ultrasound probe, the coolant is transported past electronic components that generate heat or heat sinks for such components. Once the coolant has absorbed heat inside the ultrasound probe, the heated coolant is transported back to the cable connector through the cable. At the cable connector the coolant leaves the system through an outlet. Placing the inlet and outlet of the coolant in the connector has the advantage that the potential of disinfection liquid ingress during disinfection of the probe is reduced. In general, only the probe will be subject to disinfection liquid, sometimes part of the cable, but not generally the cable connector.

As described, the inlet may connect to a first coolant reservoir, and the outlet may connect to a second coolant reservoir. The first and second coolant reservoirs may also be the same, thereby forming a closed-loop system. In this way, liquid may be drawn through the inlet to cool the ultrasound probe. For example, distilled water provides excellent cooling capabilities while reducing risks of short circuits due to its low electrical conductivity. However, such liquid cooling systems may still suffer problems arising from liquid leakage within the probe, additional erosion due to the impurity of the cooling liquid, etc. Furthermore, such liquid coolants may not always come into contact with the delicate electronics such that dedicated heat dissipation paths, e.g., a heat sink, may be necessary from which the heat is transferred to the coolant. The inventors have realized that if air is used as coolant instead, many of these drawbacks disappear, despite the fact that air is not typically used in such cooling systems owing to its lower cooling capacity as coolant. For example, air may get into contact with the electronic circuits directly reducing the need for further complicated thermal management systems. Furthermore, an air-based cooling system is more reliable and less prone to problems ranging from leakage or coolant loss. In particular, when using air as coolant, the inlet and outlet need not be connected to any particular coolant reservoir, and surrounding air may be drawn into the ultrasound cooling system through the inlet. Thereby reducing the complexity of the cooling system even further. Finally, when air surrounding the connector is drawn and hot air is expelled back to the surrounding the cooling system may be an open-loop cooling system.

Regardless of the type of coolant, according to an embodiment of the invention, coolant is drawn in through the inlet of the cable connector using a heat pump which essentially sucks in the coolant. The sucking power of the heat pump may further be adjusted based on cooling needs depending on the current state of the ultrasound probe. For example, the ultrasound probe may comprise a temperature sensor and depending on temperature measurements the heat pump intensity is determined. Similarly, depending on the current operation an expected heat generation may be determined based on which the heat pump intensity is determined. In this way, the heat pump may suck in air quicker when the ultrasound probe generates a lot of heat or is comparatively hot, while it sucks in less air when the ultrasound probe generates comparatively little heat. The intensity of the heat pump may also be made dependent on the image quality. It is known that the image quality degrades as the temperature of the transducer elements increases. In particular, it is advantageous to keep the temperature of the electronics within the ultrasound probe between 60 and 80 degrees Celsius. The heat pump may thus change intensity in order to maintain the temperature of the electronic components within said margins.

In some examples, the system may further comprise dedicated housings for components within the ultrasound probe that generate heat. These dedicated housings may in particular be configured to reduce or stop any heat dissipation within the ultrasound probe from the heat generating components to other components of the ultrasound probe. Most preferably, these dedicated housings may be configured to reduce any heat transfer from heat generating components within the ultrasound probe to ultrasound transducers of the ultrasound probe. In this way, the temperature of the ultrasound transducers can be kept lower, improving, image quality. Or in other words, the ultrasound transducers do not heat up reducing image quality.

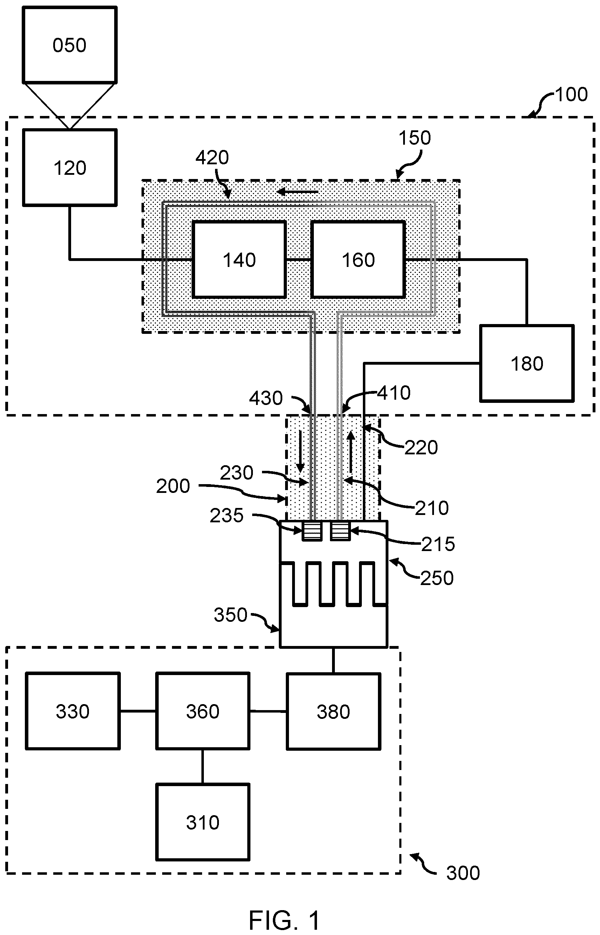

An exemplary ultrasound system implementing elements of the invention is depicted in Fig. 1. The ultrasound system according to Fig. 1 includes an ultrasound probe 100 in communication with a host 300 over a cable 200.

According to an embodiment of the invention, the ultrasound probe 100 comprises:
- an imaging sensor 120;
- an electronic circuit 150 connected to the imaging sensor, the electronic circuit configured to control the imaging sensor; and
- a cooling circuit that provides a conduit for coolant, the cooling circuit comprising:
   - an inlet 410, the inlet 410 configured to allow entry of the coolant to the cooling circuit,
   - an outlet 430, the outlet configured to allow exit of the coolant from the cooling circuit, and
   - a middle section 420 connecting the inlet of the cooling circuit to the outlet of the cooling circuit, the middle section of the cooling circuit configured to circulate the coolant past at least a part of the electronic circuit, wherein the coolant in the middle section of the cooling circuit is configured to absorb heat generated by the electronic circuit and transport the heat away. This middle section 420 may also be called a heat exchange or heat exchanger section or portion.

Fig. 1 displays an arrow within the middle section of the cooling system to indicate a flow region.

In a particularly advantageous embodiment at least a part of the electronic circuit is enclosed in the middle section 420 of the cooling circuit. In other words, a conduit is provided to circulate coolant directly past the electronic circuit 150. In such a way, the coolant may directly get into contact with the electronic circuit components. Thereby increasing the ability of the coolant to carry away heat. The middle section enclosing the electronic circuit 150 may further comprise a thermal insulation layer configured to thermally insulate the electronic circuit 150. In this way, heat generated by the electronic circuit 150 can be contained without this heat dissipating into other parts of the ultrasound probe 100. In particular, the thermal insulation layer may be configured to avoid or reduce heat dissipation from the electronic circuit 150 to the imaging sensor 120 since heat on the imaging sensor 120 can have a negative impact on image quality.

In an example, the ultrasound probe further comprises a temperature sensor determining temperature inside the ultrasound probe 100. For example, a suitable temperature sensor may be placed near the electronic circuit 150. In other examples, a temperature sensor may be placed near the imaging sensor. For example, a temperature may be inferred based on a power consumption of the ultrasound probe 100. For example, the temperature may be inferred based on an obtained image quality or an image quality decay over time. These alternatives alone or in combination allow for flexible and accurate monitoring of the temperature within the ultrasound probe. Based on the determined or inferred temperature, the cooling system settings may be adjusted. For example, the cooling system may be configured to provide more coolant or increase the coolant flow rate in order to increase the cooling capacity. In particular, based on any determined or inferred temperature or image quality decay, an intensity of a heat pump as described in more detail below may be adjusted to adjust the coolant flow rate. It is understood that the temperature sensing means or at least the processor that determines the temperature is controllably connected to the heat pump to adjust the heat pump's intensity. For example, the electronic circuit 150 of the probe may be connected with the heat pump through one or more of the plurality of connectors 210 in the cable to control the heat pump and adjust the heat pump intensity based on the measured, determined or inferred temperature. In another example, particularly when the temperature can be determined through power drawn by the probe from the host, the control of the heat pump intensity may be performed from a connection to the host 300.

The electronic circuit 150 connected to the imaging sensor may comprise a beamformer 140 and/or a processor 160. The beamformer 140 may be configured to control the imaging sensor 120, e.g., drive individual transducer elements of a plurality of transducer elements of a transducer array. The imaging sensor may be referred to in this specification as transducer array when referring particularly to an ultrasound probe. The beamformer may be any suitable type beamformer, including a microbeamformer. The electronic circuit 150 may be configured to process ultrasound signals received at the imaging sensor 120. The processor 160 may for example be a signal processor. The components of the electronic circuit 150 may be implemented in one or multiple application specific integrated circuits (ASICs) or field programmable gate arrays (FPGAs).

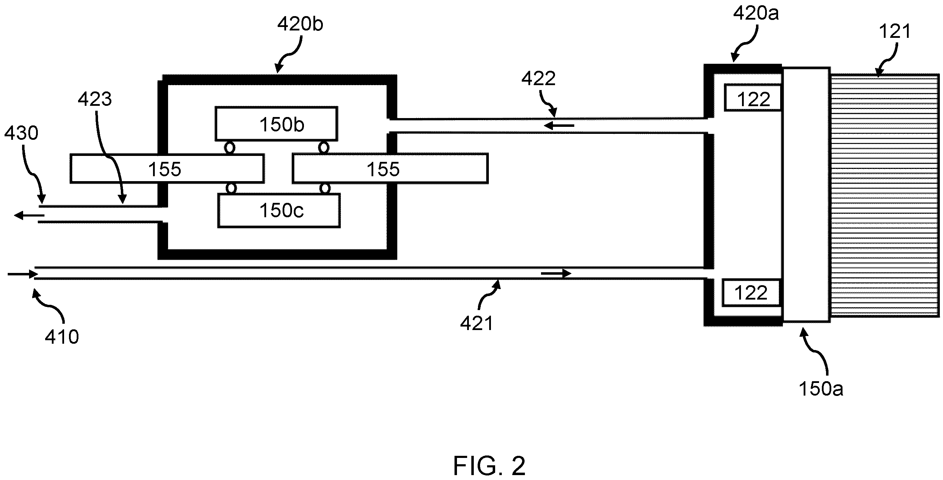

An example XMatrix ultrasound probe incorporating the embodiment of the invention as described with respect to Fig. 1 is shown in Fig. 2. In particular, Fig. 2 shows the interior set-up or arrangement of components within the housing of the XMatrix ultrasound probe. The internal components comprise:
- a transducer array 121 comprising a plurality of transducer elements;
- an electronic circuit 150a connected to the transducer array 121 and configured to control the transducer array;
- acoustic attenuation material 122;
- enclosing the electronic circuit 150a and the acoustic material is a first middle section 420a of a cooling system;
- two electronic circuits 150b and 150c;
- two printed circuit boards 155 configured to hold the two electronic circuits 150b and 150c;
- enclosing the electronic circuits 150b and 150c is a second middle section 420b of the cooling system;
- wherein the cooling system comprises:
   - an inlet 410 to allow entry of a coolant,
   - an outlet 430 to allow exit of a coolant,
   - a first coolant conduit 421 connecting the inlet to the first middle section 420a,
   - a second coolant conduit 422 connecting the first middle section 420a with the second middle section 420b, and
   - a third coolant conduit 423 connecting the second middle section 420 with the outlet 430.

The first and second middle parts 420a and 420b may further comprise a heat shield, shown in Fig. 2 by a thicker outer line. These heat shields are configured similarly as the heat shield discussed with relation to Fig. 1, namely to keep heat from dissipating into the remainder of the ultrasound probe 100.

The electronic circuits 150a, 150b, and 150c may be any type of electronic circuit. For example, electronic circuit 150a may comprise a (micro)beamformer, electronic circuits 150b and 150c may be a signal processor and an image processor, respectively. Electronic circuits 150b and 150c may also comprise a single electronic circuit. Different arrangements of electronic circuits are envisaged, and the here described arrangement is merely exemplary.

Turning back to Fig. 1, the ultrasound probe 100 may further comprise a communication interface 180.

The host 300 may include a display 310, a processor 360, a communication interface 380, and a memory 330. The host 300 and/or the processor 360 of the host 300 may also be in communication with other types of systems or devices in replacement or addition to the here mentioned systems such as for example, an external memory, external display, a subject tracking system, an inertial measurement unit, etc. It is understood that the beamformer may also be a microbeamformer. It is further understood that the components as shown here may also be configured in alternate arrangements. For example, the processor 160 and/or the (micro)beamformer 140 may be located outside of the probe 100 and/or the display 310 and/or memory 330 may be located outside of the host 300.

The ultrasound probe 100 may include a housing configured for handheld operation by a user. The transducer array 120 can be configured to obtain ultrasound data while the user grasps the housing of the probe 100 such that the transducer array 120 is positioned adjacent to or in contact with a subject's skin. The probe 100 is configured to obtain ultrasound data of anatomy within the subject's body while the probe 100 is positioned outside of the subject's body. In some embodiments, the probe 100 may also be an internal ultrasound probe such as for example a Trans Esophageal Endoscopic (TEE) ultrasound probe, an IntraVenous UltraSound (IVUS) catheter, or any other type of endoscopic or otherwise internal ultrasound probe. The subject may be a human subject, also called a patient, an animal subject, or a material subject, for example for material inspection.

The imaging sensor 120 may comprise a transducer array 120 or an array of transducers comprising a plurality of transducer elements that may emit ultrasound signals towards an object 050 of a subject and receives echo signals reflected from the object 050 back to the transducer array. The transducer array may comprise any suitable number of acoustic elements, including one or more acoustic elements and/or a plurality of acoustic elements. In some instances, the transducer array includes a single acoustic element. In some instances, the transducer array may include an array of acoustic elements with any number of acoustic elements in any suitable configuration. For example, the transducer array can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x-dimensional array (e.g., a 1.5D array), or a two-dimensional (2D) array. The array of acoustic elements (e.g., one or more rows, one or more columns, and/or one or more orientations) can be uniformly or independently controlled and activated. The transducer array can be configured to obtain one-dimensional, two-dimensional, and/or three-dimensional images of a subject's anatomy. In some embodiments, the transducer array 120 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

According to an example, the beamformer 140 may be coupled to the transducer array. The beamformer 140 controls the transducer array, for example, for transmission of the ultrasound signals and reception of the ultrasound echo signals. In some embodiments, the beamformer 140 may apply a time-delay to signals sent to individual acoustic transducers within an array in the transducer array 120 such that an acoustic signal is steered in any suitable direction propagating away from the probe 100. The beamformer 140 may further provide image signals to the processor 160 based on the response of the received ultrasound echo signals. The beamformer 140 may include multiple stages of beamforming. The beamforming can reduce the number of signal lines for coupling to the processor 160. In some embodiments, the transducer array 120 in combination with the beamformer 140 may be referred to as an ultrasound imaging component. The beamformer 140 may also be a microbeamformer.

The processor 160 is coupled to the beamformer 140. The processor 160 may also be described as a processor circuit, which can include other components in communication with the processor 160, such as a memory, beamformer 140, communication interface 180, and/or other suitable components. The processor 160, when a beamformer 140 is present, is configured to process the beamformed image signals. For example, the processor 160 may perform filtering and/or quadrature demodulation to condition the image signals. The processor 160 and/or 360 can be configured to control the transducer array 120 to obtain ultrasound data associated with the object 050.

The communication interface 180 is coupled to the processor 160. The communication interface 180 may include one or more transmitters, one or more receivers, one or more transceivers, and/or circuitry for transmitting and/or receiving communication signals. The communication interface 180 can include hardware components and/or software components implementing a particular communication protocol suitable for transporting signals over a communication link 220 inside cable 200 to the host 300. The communication link is typically composed of a plurality of conductors 220. The communication interface 180 can be referred to as a communication device or a communication interface module.

According to an embodiment of the invention, the cable 200 connects the probe 100 at a first end, and to the host 300 at a second hand. In some examples, the cable 200 may be intricately connected to the ultrasound probe 100, while being detachably connected to the host 300. Such a detachable connection may be achieved through connectors. For example, according to an embodiment of the invention, the cable comprises a cable connector 250. A host 300 configured to connect or be connected to such a cable may further comprise a host connector 350 configured to mate with the cable connector. In other words, the cable connector 250 and the host connector 350 may each comprise connection pins to enable signal transmission in order to enable a host 300 and a probe 100 to transmit signals to each other. The cable connector 250 and host connector 350 may for example comprise connectors according to the universal serial bus standard or any other connector standard. Alternatively, the connectors may be specific connectors to, for example a particular ultrasound system.

A cable 200 according to an embodiment of the invention in particular comprises:
- a jacket (i.e., the outer most layer of the cable), wherein inside the jacket the cable comprises:
   - a plurality of conductors 220 configured to transmit electrical signals from the cable connector 250 to the ultrasound probe and vice-versa; and
   - a first coolant conduit 210 configured to transport coolant from a heat pump of the cable connector 250 to the inlet 410 of the cooling circuit of the ultrasound probe 100;
   - a second coolant conduit 230 configured to transport coolant from the outlet 430 of the cooling circuit of the ultrasound probe 100 to an outlet 235 of the cable connector 250.

Arrows are displayed in Fig. 1 next to the coolant conduits 210 and 230 indicating an exemplary coolant flow direction.

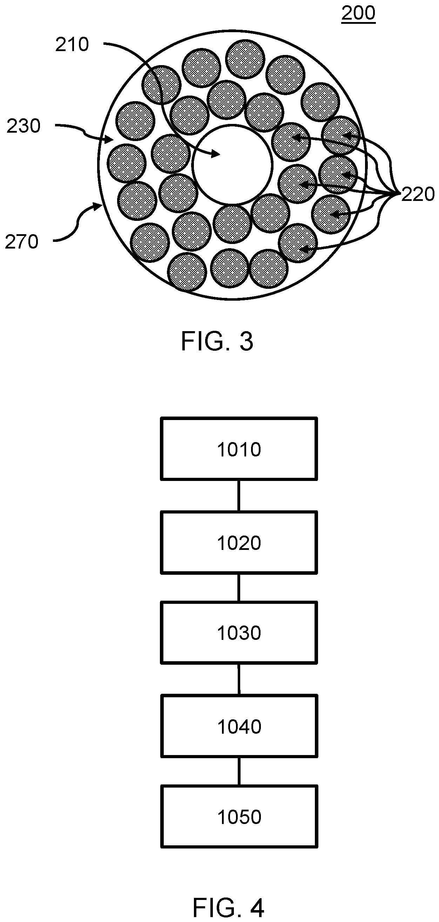

In a preferable example, the second coolant conduit 230, or at least the portion of the second coolant conduit that is within the cable, is formed by empty spaces inside the jacket between the plurality of conductors and the first coolant conduit. An example cable according to this preferable example is provided in Fig. 3.

Fig. 3 shows a cable 200 comprising a plurality of conductors 220, a first coolant conduit 210 and a second coolant conduit 230 formed by empty spaces between the jacket 270 of the cable and the plurality of conductors 220. The first coolant conduit 210 is shown in Fig. 3 to be concentrical with the jacket 270, however this must not be, and the first conduit may be arranged differently within the cable 200. Similarly, the first and second conduits may be reversed, i.e., the second conduit is used to transport coolant to the probe 100, and the first conduit is used to transport coolant away from the probe.

In an example, the cable may further be configured to comprise thermally conductive material. For example, the jacket 270 may comprise thermally conductive material. In this way, the cable may be configured to dissipate heat transported by coolant passing through the second conduit.

According to an embodiment of the invention, the cable connector 250 comprises
- a connector housing, the connector housing comprising:
   - an inlet 215, the inlet 215 configured to allow entry of a coolant to the connector housing, and
   - an outlet 235, the outlet 235 configured to allow exit of the coolant from the connector housing.

Placing the inlet and outlets of the cooling system in the connector has the advantage that no openings are placed near the probe. Disinfection methods that require, e.g., liquid disinfectants are thus not limited by this cooling system. Furthermore, the inlet and outlet need not connect to any further systems, such that for example air can be drawn into the cooling system and expelled back into the surroundings forming an open-loop cooling circuit.

In a further preferred embodiment, the cable connector further comprises
- a heat pump (not shown) inside the connector housing and connected to the inlet, the heat pump configured to draw the coolant through the inlet. However, other placements of the pump, such as within the ultrasound probe, e.g., in the middle section 420, or inside the connector housing and connected to the outlet are also envisaged herein. The placement may have an influence on the pressure distribution within the cooling circuit and the cooling capacity. Placement at the inlet is preferred to generate a positive pressure gradient over the cooling circuit and to not generate any additional heat within the ultrasound probe 100. Additional pumps may also be placed along the cooling system to improve the coolant flow.

Returning to Fig. 1, at the host 300, the communication interface 380 may receive image signals from the communication interface 180 from the probe 100 via cable 200 (e.g., via the plurality of conductors 220). The communication interface 380 may be substantially similar to the communication interface 180. The host 300 may be any suitable computing and display device, such as a workstation, a personal computer (PC), a laptop, a tablet, or a mobile phone.

The processor 360 is coupled to the communication interface 380. The processor 360 may also be described as a processor circuit, which can include other components in communication with the processor 360, such as the memory 330, the communication interface 380, and/or other suitable components. The processor 360 can be configured to generate image data from the image signals received from the probe 100. The processor 360 can apply advanced signal processing and/or image processing techniques to the image signals. An example of image processing includes conducting a pixel level analysis to evaluate whether there is a change in the color of a pixel, which may correspond to an edge of an object (e.g., the edge of an anatomical feature). In some embodiments, the processor 360 can form a three-dimensional (3D) volume image from the image data. In some embodiments, the processor 360 can perform real-time processing on the image data to provide a streaming video of ultrasound images of the object 050.

The memory 330 is coupled to the processor 360. The memory 330 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 330 may be located within the host 300. There may also be an additional external memory, or an external memory in replacement of memory 330. An external memory may be a cloud-based server or an external storage device, located outside of the host 300 and in communication with the host 300 and/or processor 360 of the host via any suitable communication link. Subject information may include measurements, data, files, other forms of medical history, such as but not limited to ultrasound images, ultrasound videos, and/or any imaging information relating to the subject's anatomy. The subject information may include parameters related to an imaging procedure such a probe position and/or orientation.

The display 310 is coupled to the processor 360. The display 310 may be a monitor or any suitable display or display device. The display 310 is configured to display the ultrasound images, image videos, and/or any imaging information of the object 050.

The system may be used to assist a sonographer or operator in performing an ultrasound scan. The scan may be performed in a point-of-care setting. In some instances, the host 300 is a console or movable cart. In some instances, the host 300 may be a mobile device, such as a tablet, a mobile phone, or portable computer. During an imaging procedure, the ultrasound system can acquire an ultrasound image of a region of interest of a subject.

It should be understood that while the ultrasound system has been described as a whole, the invention may also be split into multiple components which together form the ultrasound system. For example, an ultrasound probe 100 may be manufactured and sold separate from the cable 200 and the host 300. Similarly, the cable 200 may be sold separately from the probe 100 and the host 300. Similarly, the cable connector 250 may be sold separately as a replacement part for the cable 200. It is therefore envisaged to also seek protection for an ultrasound probe 100, a cable 200 and a cable connector 250 individually.

According to an embodiment of the invention there is provided a cable connector for an ultrasound probe and cable assembly, the cable connector comprising:
- a connector housing, the connector housing comprising:
   - an inlet, the inlet configured to allow entry of a coolant to the connector,
   - an outlet, the outlet configured to allow exit of the coolant from the connector,
   - a pump inside the connector housing and connected to the inlet, the heat pump configured to draw the coolant through the inlet and expel it through the outlet, wherein the coolant may be air, and wherein the inlet and outlet are only connected to each other via a cooling circuit through a cable as described above.

According to an embodiment of the invention there is provided an ultrasound probe, the ultrasound probe comprising:
- an imaging sensor;
- an electronic circuit configured to control the imaging sensor; and
- a cooling circuit configured to circulate coolant past at least a part of the electronic circuit, wherein the electronic circuit may be enclosed by the cooling circuit and wherein the coolant may be air.

According to an embodiment of the invention there is provided a cable for an ultrasound probe and cable assembly, the cable comprising:
- a plurality of conductors configured to transmit electrical signals;
- a first coolant conduit configured to transport coolant from a connector to an ultrasound probe; and
- a second coolant conduit configured to transport coolant from the ultrasound probe to the connector, wherein the second coolant conduit may be formed by empty spaces inside a jacket of the cable between the plurality of conductors and the first coolant conduit, and wherein the coolant may be air.

It is also possible to for example describe the cooling circuit with respect to the entire probe and cable assembly rather than only the ultrasound probe 100. In such a case, the probe and cable assembly comprises:
- an ultrasound probe (100) comprising
   - an imaging sensor (120); and
   - an electronic circuit (150) configured to control the imaging sensor;
- a cable connector (250);
- a cable (200) connecting the ultrasound probe and the cable connector, the cable comprising:
   - a plurality of conductors configured to transmit electrical signals between the cable connector and the ultrasound probe; and
- a cooling circuit comprising:
   - an inlet (215) within the cable connector, the inlet configured to allow entry of a coolant to the cooling circuit;
   - an outlet (235) within the cable connector, the outlet configured to allow exit of a coolant from the cooling circuit;
   - a heat exchanger section (420) within the ultrasound probe, the heat exchanger section configured to circulate coolant past at least part of the electronic circuit
   - a first coolant conduit (210), the first coolant conduit configured to transport coolant from the inlet through the cable to the heat exchanger section;
   - a second coolant conduit (230), the second coolant conduit configured to transport coolant from the heat exchanger section to the outlet; and
   - a pump configured to propagate coolant through the cooling system, wherein the pump may be located anywhere along the cooling circuit. This pump and any pump as described herein may be for example a fan.

As described the cooling circuit is an open-loop cooling circuit, such that when the pump propagates the coolant it also draws in coolant at the inlet and expels coolant at the outlet. List open loop and air in claim 1.

Fig. 4 displays a schematic of method of cooling an ultrasound probe according to an embodiment of the invention. The method comprises:
- drawing 1010 coolant through an inlet in a cable connector, the coolant may be drawn through the inlet in the cable connector by a heat pump;
- transporting 1020 the coolant from the cable connector to an ultrasound probe via a cable, wherein the coolant may be transported from the cable connector to the ultrasound probe via a first coolant conduit in the cable;
- circulating 1030 the coolant past at least a part of an electronic circuit inside the ultrasound probe, wherein the coolant is configured to absorb heat generated by the electronic circuit, wherein the coolant may be circulated past at least part of the electronic circuit through a cooling circuit inside the ultrasound probe;
- transporting 1040 the coolant from the ultrasound probe back to the cable connector via the cable wherein the coolant may be transported from the ultrasound probe to the cable connector via a second coolant conduit in the cable, wherein the second coolant conduit may be formed by empty spaces inside a jacket of the cable, wherein the empty spaces comprise empty spaces between a plurality of conductors inside the cable, the first coolant conduit and the jacket.; and
- expelling 1050 the coolant from the cable connector through an outlet in the cable connector.

The coolant as described in this specification with relation to all embodiments and examples may be any suitable coolant, for example liquid coolant such as water or distilled water or a gas coolant. The inventors have in particular realized that a counterintuitive coolant such as air has advantageous effects for ultrasound systems. Air would not normally be used for cooling since its cooling capacity is less than that of most liquid coolants. However, by balancing the need for cooling by limiting the power consumption in the ultrasound probe, air can provide sufficient cooling capacity, and in addition, using air reduces the chance of leakages, short circuits, and other issues inside the ultrasound probe related to liquid passing inside the probe. The inlet and outlet for the air into the cooling system are further provided within the ultrasound connector to minimize the chance of any liquid ingress during disinfection. Furthermore, by using surrounding air, no closed-loop cooling system is needed significantly reducing the complexity of the overall cooling system. In other words, the herein described cooling system provides a simple and reliable open-loop cooling solution for improving the thermal management inside an ultrasound probe.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. An ultrasound probe and cable assembly comprising:
- an ultrasound probe (100) comprising
- an imaging sensor (120); and
- an electronic circuit (150) configured to control the imaging sensor;
- a cable connector (250);
- a cable (200) connecting the ultrasound probe and the cable connector, the cable comprising:
- a plurality of conductors (220) configured to transmit electrical signals between the cable connector and the ultrasound probe; and
- an open-loop cooling circuit comprising:
- an inlet (215) within the cable connector, the inlet configured to allow entry of a coolant to the cooling circuit;
- an outlet (235) within the cable connector, the outlet configured to allow exit of a coolant from the cooling circuit;
- a heat exchanger section (420) within the ultrasound probe, the heat exchanger section configured to circulate coolant past at least part of the electronic circuit
- a first coolant conduit (210), the first coolant conduit configured to transport coolant from the inlet through the cable to the heat exchanger section;
- a second coolant conduit (230), the second coolant conduit configured to transport coolant from the heat exchanger section to the outlet; and
- a pump configured to propagate coolant through the cooling system;
wherein the coolant comprises a gas.

2. The ultrasound probe and cable assembly of claim 1,
wherein the second coolant conduit is formed by empty spaces inside a jacket (270) of the cable between the plurality of conductors and the first coolant conduit.

3. The ultrasound probe and cable assembly of any of the preceding claims,
wherein at least a part of the electronic circuit is enclosed in the heat exchanger section.

4. The ultrasound probe and cable assembly of claim 3, wherein the heat exchanger section comprises a thermal insulation layer, the thermal insulation layer configured to contain heat generated by the electronic circuit.

5. The ultrasound probe and cable assembly of any of the preceding claims,
further comprising a temperature sensor for determining a temperature inside the ultrasound probe; and
wherein an intensity of the pump is dependent on the determined temperature.

6. The ultrasound probe and cable assembly of any of the preceding claims,
wherein the cable is further configured to dissipate heat transported by the coolant in the second conduit.

7. The ultrasound probe and cable assembly of claim 6,
wherein the jacket of the cable comprises a thermally conductive material.

8. The ultrasound probe and cable assembly of any of the preceding claims,
wherein the coolant is air.

9. The ultrasound probe and cable assembly of any of the preceding claims, wherein the pump is placed at the inlet in the cable connector.

10. The ultrasound probe and cable assembly of any of the preceding claims, wherein the cooling system comprises multiple pumps.

11. A cable connector (250) for an ultrasound probe and cable assembly, the cable connector comprising:
- a connector housing, the connector housing comprising:
- an inlet (215), the inlet configured to allow entry of a coolant to the connector,
- an outlet (235), the outlet configured to allow exit of the coolant from the connector,
- a pump inside the connector housing and connected to the inlet, the heat pump configured to draw the coolant through the inlet and expel it through the outlet, wherein the coolant is a gas.

12. An ultrasound probe (100), the ultrasound probe comprising:
- an imaging sensor (120);
- an electronic circuit (150) configured to control the imaging sensor; and
- an open-loop cooling circuit configured to circulate coolant past at least a part of the electronic circuit.

13. A cable (200) for an ultrasound probe and cable assembly, the cable comprising:
- a plurality of conductors (220) configured to transmit electrical signals;
- a first coolant conduit (210) configured to transport coolant from a connector to an ultrasound probe; and
- a second coolant conduit (220) configured to transport coolant from the ultrasound probe to the connector, wherein the coolant is a gas

14. A method of cooling an ultrasound probe, the method comprising:
- drawing (1010) coolant through an inlet in a cable connector;
- transporting (1020) the coolant from the cable connector to an ultrasound probe via a cable;
- circulating (1030) the coolant past at least a part of an electronic circuit inside the ultrasound probe, wherein the coolant is configured to absorb heat generated by the electronic circuit;
- transporting (1040) the coolant from the ultrasound probe back to the cable connector via the cable;
- expelling (1050) the coolant from the cable connector through an outlet in the cable connector.

15. The method of claim 14, wherein
- the coolant is drawn through the inlet in the cable connector by a heat pump;
- the coolant is transported from the cable connector to the ultrasound probe via a first coolant conduit in the cable;
- the coolant is circulated past at least part of the electronic circuit through a cooling circuit inside the ultrasound probe; and
- the coolant is transported from the ultrasound probe to the cable connector via a second coolant conduit in the cable.
